(19) Europäisches Patentamt
European Patent Office
Office européen des brevets

(11) Publication number: **0 156 634**
**B1**

(12) **EUROPEAN PATENT SPECIFICATION**

(45) Date of publication of patent specification: **03.08.88**

(21) Application number: **85302011.3**

(22) Date of filing: **22.03.85**

(51) Int. Cl.[4]: **B 01 J 29/06,** B 01 J 29/14, B 01 J 29/24, B 01 J 29/34, C 07 C 2/12, C 07 C 1/26, C 07 C 17/154

(54) **Catalyst for oxyhalogenation and condensation of alkanes.**

(30) Priority: **29.03.84 US 594583**

(43) Date of publication of application: **02.10.85 Bulletin 85/40**

(45) Publication of the grant of the patent: **03.08.88 Bulletin 88/31**

(84) Designated Contracting States: **BE DE FR GB IT NL**

(56) References cited:
**EP-A-0 106 356**
**FR-A-2 231 644**
**GB-A-1 263 806**
**US-A-3 914 328**
**US-A-4 297 328**

(73) Proprietor: **MOBIL OIL CORPORATION**
**150 East 42nd Street**
**New York New York 10017 (US)**

(72) Inventor: **Chu, Pochen**
**1173 Ollerton Road**
**West Deptford New Jersey 08066 (US)**
Inventor: **Dwyer, Francis Gerard**
**1128 Talleyrand Road**
**West Chester Pennsylvania 19380 (US)**

(74) Representative: **Cooper, John Anthony et al**
**Mobil Court 3 Clements Inn**
**London WC2A 2EB (GB)**

**EP 0 156 634 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a composite catalyst for converting natural gas and other alkane feedstocks into higher hydrocarbons, such as gasoline blending stocks and petrochemical materials.

The relative abundance of methane in natural gas and the high cost of petroleum raw materials has led workers in the field of fossil fuel technology to seek economic processes for the conversion of lower alkanes into higher hydrocarbons in the gasoline and diesel boiling ranges. The $C_5^+$ aliphatic and aromatic hydrocarbons are particularly useful as liquid fuels for spark ignition internal combustion engines. Steam reforming of methane-rich feedstocks to produce CO and $H_2$ (syngas) can be followed by conventional methanol synthesis or Fischer-Tropsch reactions to provide oxygenated feedstock suitable for hydrocarbon conversion over catalysts comprising crystalline, medium pore zeolites, such as ZSM-5 zeolite.

A substantial effort has been made in developing a methanol-to-gasoline ("MTG") process, as described in U.S.—A—3,894,107, U.S.—A—4,013,731, U.S.—A—4,118,431, and U.S.—A—4,251,484. Other processes involving the use of ZSM-5 type zeolites for converting synthesis gas into gasoline-range hydrocarbons are described in U.S.—A—4,159,995 and U.S.—A—4,093,029.

A dual function composite catalyst has been devised comprising an intimate mixture of a Deacon catalyst and a medium pore acid zeolite. This catalyst can be employed in an integrated process for halogenating alkanes and condensing the haloalkane intermediate to produce higher hydrocarbons. In a preferred embodiment, the dual-function catalyst comprises a Deacon catalyst deposited on alumina and composited with HZSM-5 crystals. Typical Deacon catalysts include $CuCl_2$, alkali metal chloride or alkaline earth metal chloride, and rare earth chloride impregnated on a solid carrier.

Halohydrocarbons can be made from lower aliphatic compounds by a number of halogenation processes. Emphasis is placed herein on the chlorination of methane and other components of natural gas as a preferred economic process for making higher hydrocarbons. $C_1$ to $C_3$ paraffins are abundant in nature, frequently occurring with carbon dioxide and trace amounts of $C_4$+ hydrocarbons. A typical dry gas feedstock may contain 90+ vol % $CH_4$ and lesser amounts of $CO_2$ and ethane.

A suitable halogenation gas may include molecular chlorine ($Cl_2$) or hydrogen chloride (HCl) with oxygen. It is well known that halogen gases may be generated by heating hydrogen halide in contact with an oxygen-containing gas, such as air. When combined with heated methane over a Deacon catalyst, a typical halogenation reaction occurs as follows:

$$CH_4+HCl+1/2\ O_2 \rightarrow CH_3Cl+H_2O$$

Deacon catalysts are well-known materials and are commonly used in halogenation reactions, especially where HCl is oxidized as part of the reaction. Although catalyst melts are known, particulate catalysts such as obtained by impregnating cupric halide on calcined alumina are preferred, especially those Deacon catalysts which consist essentially of cupric chloride, alkali metal chloride and rare earth chloride calcined on alumina. Preparation of suitable catalysts is described in U.S.—A—3,184,515, U.S.—A—3,634,330 and U.S.—A—4,323,716. Halogenation processes are described in U.S.—A—2,957,924, U.S.—A—3,314,760, U.S.—A—1,654,821 and U.S.—A—4,199,533.

In U.S.—A—2,488,083 such an oxyhalogenation first stage is combined with a catalytic second stage condensation to produce higher hydrocarbons, including $C_2$—$C_4$ paraffins and olefins and $C_5^+$ aliphatics and aromatics. The catalysts involved include alumina, silica and related condensation catalysts. The second stage dehydrohalogenation reaction may be simplified as follows:

$$(n)\ CH_3Cl \rightarrow [HC]+nHCl.$$

It is recognized that the hydrocarbon products (HC) may include light gases such as ethane, propane, propylene and butanes, as well as the condensed liquid hydrocarbon products and halogenated by-products.

A process for converting aliphatic halides using ZSM-5 type zeolites is described in U.S. Patent 3,894,107. The preferred catalysts comprise crystalline aluminosilicates having a silica to alumina ratio of at least 12 and a constraint index of 1 to 12. In the acid form these are often denominated HZSM-5 type materials. Co-extruded with alumina, these active zeolites are available in various forms and sizes such as 1.6 mm cylindrical extrudate particles.

The zeolites that are useful as components of the composite catalysts of the invention are those having a silica to alumina mole ratio greater than 12:1, preferably greater than 30:1, and a constraint index of 1 to 12, and are exemplified by the shape-selective, medium-pore, siliceous zeolites ZSM-5, ZSM-5/ZSM-11 intermediate, ZSM-11, ZSM-12, ZSM-22, ZSM-23, ZSM-35, ZSM-38 and ZSM-48.

Of those zeolites, ZSM-5 is described in U.S.—A—3,702,886, Re. 29,948 and U.S.—A—4,061,724; ZSM-5/ZSM-11 intermediate is described in U.S.—A—4,229,424; ZSM-11 is described in U.S.—A—3,709,979; ZSM-12 is described in U.S.—A—3,832,449; ZSM-23 is described in U.S.—A—4,076,842; ZSM-35 is described in U.S.—A—4,016,245; ZSM-38 is described in U.S.—A—4,046,859; and ZSM-48 is described in EP—A—00 15 132.

Such zeolites may be used in the catalysts of the invention in the hydrogen form or they may be base

exchanged or impregnated to contain a metal cation. It is desirable to calcine the zeolite after base exchange.

The catalyst compositions of the invention may be prepared in various ways. For example, the components may be separately prepared in the form of particles such as pellets or extrudates, and intimately mixed in the required proportions. The sizes of the individual component particles may be quite small, or they may be as large as up to about 13 mm. Alternatively, the components may be mixed as powders and formed into composite pellets or extrudate, each pellet containing both components in substantially the required proportions.

It is desirable to incorporate the zeolite component of the composition in a matrix. Such a matrix is useful as a binder and imparts greater resistance to the catalyst towards severe temperature, pressure and velocity conditions. Hydrated alumina ($\gamma$-$Al_2O_3 \cdot H_2O$) is a preferred zeolite binder. Other matrix materials include both synthetic and natural substances. Such substances include clays, silica and/or metal oxides. The latter may be either naturally occurring or in the form of gelatinous precipitates, sols or gels including mixtures of silica and metal oxides. Frequently, zeolite materials have been incorporated into naturally occurring clays, for example, bentonite and kaolin. In addition, the zeolite may be composited with a porous matrix material such as silica-alumina, silica-magnesia, silica-zirconia, silica-thoria, silica-beryllia, silica-titania, as well as ternary compositions such as silica-alumina-thoria, silica-alumina-zirconia, silica-alumina-magnesia and silica-magnesia-zirconia. The matrix can be in the form of a co-gel. A mixture of clay in combination with silica or any of the above specified co-gels may be used to form a matrix.

In general, the crystalline zeolites are ordinarily ion exchanged with a desired cation to replace alkali metal present in the zeolite as found naturally or as synthetically prepared. The exchange treatment is such as to reduce the alkali metal content to less than about 50% by weight of the original alkali metal contained in the zeolite as synthesized, usually 0.5 weight percent or less.

The purpose of ion exchange is to substantially remove alkali metal cations which are known to be deleterious to catalytic activity, as well as to introduce particularly desired catalytic activity by means of the various cations used in the exchange medium. For the hydrocarbon conversion operation described herein, preferred cations are hydrogen, ammonium, rare earth and mixtures thereof, with particular preference being accorded to rare earth. Ion exchange is suitably accomplished by conventional contact of the zeolite with a suitable salt solution of the desired cation such as, for example, the sulfate, chloride or nitrate.

The ZSM-5 type zeolite used in the catalysts of the invention has the particular advantage of a hydrophobic nature and resistance to halogen acid deterioration. These characteristics are not readily found in known condensation catalysts. By eliminating interstage separation of corrosive inorganic halogen materials and water from the haloalkanes, an economic process is achieved.

In general, conversion of alkyl halides to useful hydrocarbon product takes place in the temperature range of 300 to 500°C in the presence or absence of hydrogen. Pressures from below atmospheric to about 7000 kPa may be used, those of 5 to 500 kPa being preferred. Typically, a weight hourly space velocity of about 0.5 to 50 WHSV based on zeolite content is employed in the catalyst bed.

The various products may be recovered or recycled according to process needs. Unconverted methane and other light gases may be separated and recycled to the catalyst zone. Inorganic halogen-containing gases may be separated for recycle, regeneration or disposal, according to their composition. Halohydrocarbons may be recovered as product or recycled.

The following Examples illustrate the invention.

Example 1

A Deacon chlorination catalyst was prepared by impregnating 30 parts by weight of a low surface area (14 $m^2/g$) and medium porosity (0.25 ml/g) alumina with a solution of 6.1 parts by weight of $CuCl_2 . 2H_2O$, 4.2 parts by weight of KCl and 6.4 parts by weight of $RECl_3$ in 60 parts by weight of water; it was then dried at 110°C and calcined at 371°C for 3 hours. An acid ZSM-5 was prepared by calcining the "as synthesized" ZSM-5 ($SiO_2/Al_2O_3$=40) at 538°C in $N_2$ for 3 hours. The calcined zeolite was ion exchanged with $NH_4NO_3$ solution to reduce Na to less than 0.02% by weight, then converted into acid form by air calcination at 538°C for 3 hours. One part of chlorination catalyst and four parts of acid ZSM-5 were then composited together.

Example 2

A feedstock of methane and chlorine was reacted over the composite catalyst of Example 1 in a downflow tubular reactor. The operating conditions and results are shown in Table I.

TABLE I
Catalyst: Chlorination and ZSM-5 catalyst composite
Total weight: 5 grams

| Run No. | 2A | 2B | 2C |
|---|---|---|---|
| Feedstock | | | |
| Methane, ml/min | 23.5 | 23.5 | 23.5 |
| Chlorine, ml/min | 5.0 | 5.0 | 5.0 |
| Operating conditions | | | |
| Temperature, °C | 427 | 427 | 454 |
| Pressure, kPa | 100 | 100 | 100 |
| Time on stream, hours | 1.5 | 3.0 | 1.0 |
| $CH_4$ Conversion, mol % | 6.09 | 7.3 | 5.08 |
| Product distribution, mol % | | | |
| $C_1$ | 93.91 | 92.70 | 94.92 |
| $C_2$ | 0.46 | 1.14 | 1.48 |
| $C_3$ | 4.83 | 3.89 | 1.83 |
| $C_4^+$ | 0.82 | 2.26 | 1.77 |

Example 3

A Deacon chlorination catalyst was prepared by impregnating 100 parts by weight of particles of calcined alumina (Alcoa A-3) with 6.1 parts by weight of $CuCl_2 . 2H_2O$, 2.7 parts by weight of $MgCl_2$ and 6.2 parts by weight of rare earth chloride in 61 parts by weight of water. The impregnated particles were dried at about 100°C to remove water and then calcined at about 370°C for 3 hours. This catalyst was mixed with HZSM-5 as described in Example 1, in the proportions described in Example 1.

Example 4

A Deacon oxychlorination catalyst was prepared by impregnating 100 parts by weight of particles of calcined alumina (Alcoa A-3) with 10.1 parts by weight $CuCl_2 . 2H_2O$, 7.0 parts by weight KCl, and 10.6 parts by weight rare earth chloride dissolved in 100 parts by weight of water. The impregnated particles were dried at about 110°C to remove water and calcined at about 370°C for three hours. The Deacon catalyst was then mixed with a standard commercially available acid ZSM-5 catalyst containing 65 wt.% ZSM-5 and 35% alumina in 1.6 mm extrudate form. The composite catalyst contained four parts by weight of ZSM-5 extrudate and one part by weight of Deacon catalyst.

The Deacon catalyst components may be deposited on a zeolite condensation catalyst as carrier. Typically, the active halogenation components comprise about 1 to 20 weight percent of the active zeolite, with about 3 to 12 wt.% being preferred. Typically, a fixed bed catalyst comprises about 10 to 90% active catalytic components, the remainder being inert binder.

In the oxyhalogenation of alkanes, it is advantageous to control the relative amounts of reactant gases to minimize formation of polyhalogenated alkanes, such as methylene chloride ($CH_2Cl_2$), chloroform and dihalo ethanes, for example. This may be achieved in a continuous process by regulating the flow of hydrocarbon gas to maintain a stoichiometric excess relative to the halogen. Ordinarily, the mole ratio of alkane to halogen is in the range of 1:1 to 10:1. To avoid deleterious effects in the system, it may be desirable to limit halogen-acid gas concentration, especially where gases such as HCl or HBr could combine with water.

Direct halogenation of lower alkanes is a well-known industrial process. In the absence of light or catalyst, thermal chlorination of methane is carried out as a chain reaction by thermal dissociation of $Cl_2$. A lower activation energy is required for catalytic chlorination. Direct chlorination may be depicted by the following:

$$CH_4+Cl_2 \rightarrow CH_3Cl+HCl.$$

Both direct chlorination and oxychlorination are believed to proceed by generating chlorine atoms, which react with methane to form HCl and a methyl radical. The $CH_3$ radical in turn reacts with chlorine to form chloromethane.

The addition of oxygen to halogenation gases is well known. Hydrogen halides (HX) are converted to halogen and water by heating with an oxygen-containing gas such as air or pure $O_2$. Where oxygen is maintained in a stoichiometric excess to HX, little if any hydrogen halide gas will escape the halogenation zone. For this reason, oxyhalogenation is advantageous as compared to direct halogenation. Where $O_2$ and $Cl_2$ are present in the halogenation gas, HCl formed is reacted with alkane and $O_2$ for further conversion.

**Claims**

1. A catalyst composition comprising (a) a Deacon catalyst comprising cupric halide, alkaline earth metal halide and/or alkali metal halide, and/or rare earth metal halide and (b) a crystalline aluminosilicate zeolite having a silica to alumina mole ratio greater than 12:1 and constraint index of 1 to 12.

2. A catalyst composition according to claim 1, wherein the Deacon catalyst component comprises from 1 to 20 weight percent of the zeolite component.

3. A catalyst composition according to claim 1 or claim 2, which comprises from 10 to 90 weight percent of an alumina binder.

4. A catalyst composition according to any one of claims 1 to 3, wherein the zeolite component is acid ZSM-5 having a silica:alumina mole ratio greater than 30:1.

5. A catalyst composition according to any one of claims 1 to 4, wherein the Deacon catalyst components are deposited on alumina and composited with HZSM-5.

6. A catalyst composition according to any one of claims 1 to 5, wherein the Deacon catalyst includes $CuCl_2$, alkali metal chloride or alkaline earth chloride, and rare earth chloride, impregnated on alumina.

**Patentansprüche**

1. Katalysatorzusammensetzung, die (a) einen Deacon-Katalysator, der Kupferhalogenid, ein Erdalkalimetallhalogenid und/oder Alkalimetallhalogenid und/oder ein Halogenid eines Metalls der Seltenen Erden umfaßt und (b) einen kristallinen Aluminosilicatzeolith umfaßt, der ein Molverhältnis von Siliziumdioxid zu Aluminiumoxid von größer als 12:1 und einen Zwangsindex von 1 bis 12 aufweist.

2. Katalysatorzusammensetzung nach Anspruch 1, worin die Deacon-Katalysatorkomponente von 1 bis 20 Gew.-% der Zeolithkomponente umfaßt.

3. Katalysatorzusammensetzung nach Anspruch 1 oder 2, die von 10 bis 90 Gew.-% eines Aluminiumoxidbindemittels umfaßt.

4. Katalysatorzusammensetzung nach einem der Ansprüche 1 bis 3, worin die Zeolithkomponente ein saurer ZSM-5 mit einem Siliziumdioxid/Aluminiumoxid-Molverhältnis von größer als 30:1 ist.

5. Katalysatorzusammensetzung nach einem der Ansprüche 1 bis 4, worin die Deacon-Katalysatorkomponenten auf Aluminiumoxid abgeschieden sind und mit HZSM-5 zusammengesetzt sind.

6. Katalysatorzusammensetzung nach einem der Ansprüche 1 bis 5, worin der Deacon-Katalysator $CuCl_2$, ein Alkalimetallchlorid oder ein Erdalkalimetallchlorid und ein Chlorid der Seltenen Erden umfaßt, die auf Aluminiumoxid imprägniert werden.

**Revendications**

1. Une composition de catalyseur comprenant:

(a) un catalyseur de Deacon comprenant halogénure cuivrique, un halogénure de métal alcalino terreux et/ou un halogénure de métal alcalin et/ou un halogénure de métal de terres rares; et

(b) une zéolite d'aluminosilicate cristallin dont le rapport molaire silice/alumine est supérieur à 12/1 et l'indice de contrainte est compris entre 1 et 12.

2. Une composition de catalyseur selon la revendication 1, dans laquelle le catalyseur de Deacon constitue de 1 à 20% du composant zéolitique.

3. Une composition de catalyseur selon la revendication 1 ou la revendication 2, qui comprend de 1 à 90% en poids d'un liant à base d'alumine.

4. Une composition de catalyseur selon l'une quelconque des revendications 1 à 3, dans laquelle le composant zéolitique est une ZSM-5 acide dont le rapport molaire silice/alumine est supérieur à 30/1.

5. Une composition de catalyseur selon l'une quelconque des revendications 1 à 4, dans laquelle les composants du catalyseur de Deacon sont déposés sur de l'alumine et formulés avec de la ZSM-5.

6. Une composition de catalyseur selon l'une quelconque des revendications 1 à 5, dans laquelle le catalyseur de Deacon comprend $CuCl_2$, un chlorure de métal alcalin et un chlorure de métal alcalino-terreux et un chlorure de métal de terres rares, imprégnés sur de l'alumine.